# FASCICULE DE BREVET EUROPEEN

(11) **EP 1 319 393 B1**
(45) Date de publication et mention de la délivrance du brevet: **02.09.2009**
(21) Numéro de dépôt: 02293101.8
(22) Date de dépôt: 16.12.2002
(51) Int. Cl.: A61K 8/34, A61K 8/66, A61Q 5/10

(54) **Composition de teinture des fibres kératiniques contenant une alcool oxydase et procédé mettant en oeuvre cette composition**
Zusammensetzung zur Färbung von keratinischen Fasern mit einer Alkoholoxidase und Verfahren zur Anwendung derselben
Composition for dyeing keratin fibres with an alcohol oxidase and process for applying this composition

(30) Priorité: 17.12.2001 FR 0116317
(43) Date de publication de la demande: 18.06.2003
(73) Titulaire: L'ORÉAL, 75008 Paris (FR)
(72) Inventeur: Burgaud, Hervé, 77230 Damartin en Goele (FR); Pereira, Rui, 77140 Montevrain (FR)
(74) Mandataire: Casalonga, Axel

(56) Documents cités:
- WO-A-02/47633
- DE-A- 10 016 279
- DE-A- 10 057 532
- DE-A- 10 057 545
- US-A- 5 538 517
- US-A- 5 849 041
- TSUJINO Y. ET AL: 'Hair coloring and waving using oxidases.' J. SOC. COSMET. CHEM. vol. 42, 1991, pages 273 - 282, XP002055535

## Description

La présente invention a pour objet une composition pour la teinture des fibres kératiniques contenant au moins un précurseur de colorant d'oxydation, telle qu'elle comprend en outre au moins une enzyme alcool oxydase en tant que seule enzyme de la composition et au moins un substrat pour ladite enzyme.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi la demande de brevet FR 2769219 décrit l'utilisation d'une enzyme uricase et de son substrat l'acide urique en coloration d'oxydation pour la teinture de fibres kératiniques. La demande EP-A-0 310 675 décrit l'utilisation de précurseur de colorant d'oxydation de type benzénique en association avec des enzymes telles que la pyranose-oxydase, la glucose-oxydase. Se pose aussi parfois le problème de manque de solubilité du donneur ce qui est notamment le cas de l'acide urique qui est le donneur de l'uricase.

Le but de la présente invention est de fournir de nouvelles compositions pour la teinture des fibres kératiniques par coloration d'oxydation qui respectent la nature de la fibre kératinique et qui ne présentent pas les problèmes de solubilisation et de cristallisation rencontrés notamment avec le système acide urique/ uricase.

De manière avantageuse et inattendue, la demanderesse vient maintenant de découvrir qu'il est possible d'atteindre ce but en utilisant, en tant que seule enzyme de la composition, au moins une enzyme de type alcool oxydase selon la présente revendication 1 et un substrat pour ladite enzyme dans une composition pour la teinture des fibres kératiniques, et en particulier des fibres kératiniques humaines telles que les cheveux.

La composition selon l'invention contient, dans un support approprié pour la teinture, au moins un précurseur de colorant d'oxydation, cette composition étant telle qu'elle comprend au moins une enzyme alcool oxydase en tant que seule enzyme de la composition choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7), et au moins un substrat pour ladite enzyme.

Les compositions selon la présente invention permettent l'obtention de teintures de couleurs puissantes, peu sélectives et résistantes, ces compositions sont capables d'engendrer de nouveaux colorants puissants pouvant donner des nuances variées de couleur intense et uniforme, sans dégradation signifiante du cheveu. En outre, on a noté que l'utilisation d'une telle composition améliore la tenue des cheveux permanentés et diminue la porosité du cheveu.

Dans le cadre de la présente invention, les enzymes alcool oxydases utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes sont choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro*, modifiées ou non modifiées génétiquement, ou synthétiques (obtenues par synthèse chimique ou biotechnologique).

A titre d'exemples on peut citer en particulier les genres *Pinus, Gastropode, Manduca, Pichia, Candida, Pleurotus, Pseudomonas*, et de façon encore plus particulière les espèces suivantes : *Pinus strobus* qui est une espèce d'origine végétale, *Gastropode mollusc, Manduca sexta* qui sont d'origine animale, *Pichia pastoris* et *Candida boidinii* qui sont des levures, *Pleurotus pulmonarius* qui est un champignon, et *Pseudomonas pseudoalcaligenes* qui est une bactérie.

Généralement, la concentration en enzyme alcool oxydase utilisée dans la composition tinctoriale est comprise 0,005% et 40% en poids par rapport au poids total de ladite composition et de préférence comprise entre 0,05% et 10% en poids par rapport au poids de cette composition.

L'activité enzymatique des enzymes alcool oxydases utilisées conformément à l'invention peut être définie à partir de l'oxydation du donneur en condition aérobie. Unité U correspond à la quantité d'enzyme conduisant à la génération de 1 µmole de peroxyde d'hydrogène par minute à un pH donné et à une température de 25°C.

De façon préférentielle la quantité d'alcool oxydase est comprise entre 0,2U et 4.10⁸ unités U pour 100g de composition tinctoriale.

Le ou les substrats pour ladite enzyme, encore appelés donneurs pour l'enzyme, utilisés dans les compositions selon l'invention sont choisis parmi les alcools et les précurseurs de colorants d'oxydation portant au moins une fonction alcool.

D'un point de vue pratique, le substrat pour l'enzyme est généralement couramment contenu dans les compositions pour la teinture d'oxydation des fibres kératiniques de l'art antérieur soit en tant que précurseur de colorant d'oxydation, soit en tant que solvant ou conservateur lorsqu'il s'agit d'un alcool.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Selon une première variante, préférée, le substrat pour l'enzyme est un alcool. La nature de ce substrat varie en fonction de la nature de l'enzyme alcool oxydase qui est utilisée.

Ce substrat pour l'enzyme sera de préférence un alcool choisi parmi les alcools primaires, secondaires, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4- méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Généralement, la concentration en alcool est comprise entre 0,01% et 20% en poids par rapport au poids total de la composition et de préférence comprise entre 0,05% et 10% en poids par rapport au poids total de la composition.

La présence d'au moins un précurseur de colorant d'oxydation classique est obligatoire lorsque, selon la variante préférée, la composition tinctoriale contient un alcool en tant que substrat.

Ce précurseur classique est optionnel lorsque la composition selon l'invention contient un précurseur de colorant d'oxydation portant une fonction alcool.

La nature des précurseurs de colorants (bases et/ou coupleurs) d'oxydation classiques utilisés dans la composition selon l'invention n'est pas critique.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2-hydroxy 4,5,6-triaminopyrimidine, la 2,4-dihydroxy 5,6-diaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Généralement la concentration de cette base d'oxydation est comprise entre 0,0005 et 12% en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration de ce coupleur d'oxydation est comprise entre 0,0001 et 10% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition utilisables dans le cadre de l'invention sont par exemple choisis parmi les sels d'addition avec la soude, la potasse, l'ammoniaque, les amines ou les alcanolamines.

Selon une deuxième variante de l'invention, le substrat pour l'enzyme est un précurseur de colorant d'oxydation classique portant une fonction alcool.

Dans ce cas, la présence d'un autre précurseur de colorant d'oxydation classique ne portant pas de fonction alcool n'est pas nécessaire. La composition selon l'invention peut comprendre, dans un support approprié pour la teinture, un système colorant constitué d'au moins un précurseur de colorant d'oxydation portant au moins une fonction alcool et au moins une enzyme alcool oxydase.

Ledit précurseur sera alors choisi parmi les bases d'oxydation portant au moins une fonction alcool suivantes les paraphénylènediamines, les bis-phénylalkylènediamines, les para-aminophénols, les ortho-aminophénols, les bases hétérocycliques et leurs sels d'addition, et les coupleurs d'oxydation portant au moins une fonction alcool suivants les métaphénylènediamines, les métaaminophénols, les métadiphénols, les coupleurs naphtaléniques et les coupleurs hétérocycliques ainsi que leurs sels d'addition.

Par base ou coupleur portant au moins une fonction alcool au sens de la présente invention, on entend un composé appartenant à l'une des familles citées ci-dessus, comprenant au moins une chaîne hydrocarbonée, saturée ou insaturée, linéaire ou ramifiée, comprenant de 1 à 18 et de préférence de 1 à 12 atomes de carbone substituée par au moins un groupe OH, de préférence substituée par un à quatre groupes OH.

De manière préférée, ce précurseur sera choisi parmi les bases d'oxydation suivantes la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la N-(β-hydroxypropyl) paraphénylènediamine, la 2-hydroxyméthyl paraphénylènediamine, la N,N-(éthyl, β-hydroxyéthyl) paraphénylènediamine, la N-(β,γ-dihydroxypropyl) paraphénylènediamine, la 2-β-hydroxyéthyloxy paraphénylènediamine, le 2-β hydroxyéthylamino 5-amino toluène, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(β-hydroxyéthyl) N,N'-bis-(4-aminophényl) tétraméthylènediamine, le 4-amino 3-hydroxyméthyl phénol, le 4-amino 2-hydroxyméthyl phénol, le 4-amino 2-(β-hydroxyéthyl aminométhyl) phénol, le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, le 4,5-diamino 1-(β-hydroxyéthyl) pyrazole, le 4,5-diamino 1-(β-hydroxyéthyl) 3-méthyl pyrazole, le 4,5-diamino 3-hydroxyméthyl 1-méthyl pyrazole, le 4,5-diamino 3-hydroxyméthyl 1-isopropyl pyrazole et les coupleurs d'oxydation suivants le 5-N-(β-hydroxyéthyl)amino 2-méthyl phénol, le 2,4-diamino 1-(β-hydroxyéthyloxy) benzène, le 2-amino 4-(β-hydroxyéthylamino) 1-méthoxybenzène, le 1-β-hydroxyéthylamino-3,4-méthylènedioxybenzène.

Lorsque le substrat est une base d'oxydation portant une fonction alcool, la concentration de cette base est comprise entre 0,0005 et 12 % en poids par rapport au poids total de la composition.

Lorsque le substrat est un coupleur d'oxydation portant une fonction alcool, la concentration de ce coupleur est comprise entre 0,0001 et 10 % en poids par rapport au poids total de la composition.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, méthiniques, azométhiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 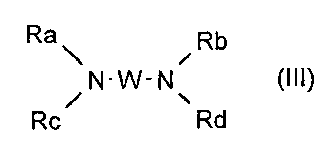 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, la durée de cette application étant la durée suffisante pour développer la coloration désirée.

La couleur est alors révélée par l'oxygène de l'air ou à l'aide d'un agent oxydant.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase et au moins un substrat pour ladite enzyme, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, puis à procéder au mélange des compositions (A) et (B) au moment de l'emploi avant d'appliquer ce mélange sur les fibres kératiniques.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention.

### EXEMPLES

### Exemple 1

On prépare la composition suivante

| | |
|---|---|
| Paraphénylènediamine ( base d'oxydation) | 3,2 g |
| Résorcinol (coupleur) | 3,3 g |
| Alcool oxydase de *Pichia pastoris* 5000U | 2 g |
| Ethanol (donneur) | 4 g |
| Tampon phosphate 0,1M dans l'eau à pH 9 | qsp100g |

On applique la composition ci-dessus sur des mèches de cheveux gris naturels à 90% de blancs et on laisse poser pendant 30 minutes. Les cheveux ont ensuite été rincés, lavés avec un shampooing standard, puis séchés.

### Exemple 2

| | |
|---|---|
| Para-aminophénol ( base d'oxydation) | 3,3 g |
| 2-méthyl 5-aminophénol (coupleur) | 3,7 g |
| Alcool oxydase de *Pichia pastoris* 5000U | 2 g |
| Ethanol (donneur) | 4 g |
| Tampon phosphate 0,1M dans l'eau à pH 9 | qsp100g |

On applique la composition ci-dessus sur des mèches de cheveux gris naturels à 90% de blancs et on laisse poser pendant 30 minutes. Les cheveux ont ensuite été rincés, lavés avec un shampooing standard, puis séchés.

### Exemple 3

| | |
|---|---|
| Paraphénylènediamine (base d'oxydation) | 3,2 g |
| Résorcinol (coupleur) | 3,3 g |
| Alcool oxydase de *Candida boidinii* 2500U | 1 g |
| Ethanol (donneur) | 4 g |
| Tampon phosphate 0,1M dans l'eau à pH 9 | qsp100g |

On applique la composition ci-dessus sur des mèches de cheveux gris naturels à 90% de blancs et on laisse poser pendant 30 minutes. Les cheveux ont ensuite été rincés, lavés avec un shampooing standard, puis séchés.

### Exemple 4

| | |
|---|---|
| Para-aminophénol + paraphénylènediamine (bases d'oxydation) | 3,2 g 3,3 g |
| Résorcinol (coupleur) | 3,3 g |
| alcool aromatique oxydase de *Pleurotus pulmonarius* 500U | 2 g |
| Alcool benzylique (donneur) | 5 g |
| Tampon phosphate 0,1M dans l'eau à pH 7 | qsp100g |

On applique la composition ci-dessus sur des mèches de cheveux gris naturels à 90% de blancs et on laisse poser pendant 30 minutes. Les cheveux ont ensuite été rincés, lavés avec un shampooing standard, puis séchés.

### Exemple 5

| | |
|---|---|
| paraphénylènediamine (base d'oxydation) | 3,2 g |
| 2- hydroxyethyloxyorthophénylènediamine (coupleur et donneur) | 5,8 g |
| alcool aromatique oxydase de *Pleurotus pulmonarius* 500U | 2 g |
| Tampon phosphate 0,1M dans l'eau à pH 7 | qsp100g |

On applique la composition ci-dessus sur des mèches de cheveux gris naturels à 90% de blancs et on laisse poser pendant 30 minutes. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| 1 | Châtain foncé |
| 2 | Cuivré |
| 3 | Châtain clair |
| 4 | Châtain |
| 5 | Bleu violacé |

## Revendications

1. Composition pour la teinture des fibres kératiniques, et en particulier les fibres kératiniques humaines telles que les cheveux, contenant, dans un support approprié pour la teinture, au moins un précurseur de colorant d'oxydation, **caractérisée par le fait qu'**elle comprend au moins une enzyme alcool oxydase en tant que seule enzyme de la composition choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) et au moins un substrat pour ladite enzyme.

2. Composition selon la revendication 1, telle que l'enzyme alcool oxydase est issue d'un extrait choisi parmi les extraits de végétaux, d'animaux ou de microorganismes (bactérie, champignon, levure, microalgue ou virus), de cellules différenciées ou dédifférenciées, obtenues *in vivo* ou *in vitro*, modifiées ou non modifiées génétiquement, ou synthétiques.

3. Composition selon la revendication 2, telle que l'enzyme alcool oxydase est choisie parmi les genres *Pinus, Gastropode, Manduca, Pichia, Candida, Pleurotus, Pseudomonas*, et de façon encore plus particulière parmi les espèces suivantes : *Pinus strobus*, *Gastropode mollusc, Manduca sexta, Pichia pastoris, Candida boidinii, Pleurotus pulmonarius* et *Pseudomonas pseudoalcaligenes*.

4. Composition selon l'une des revendications 1 à 3, telle que la concentration en enzyme alcool oxydase est comprise entre 0,005% et 40% en poids par rapport au poids total de ladite composition et de préférence comprise entre 0,05% et 10% en poids par rapport au poids total de ladite composition.

5. Composition selon l'une des revendications 1 à 4 telle que le substrat pour l'enzyme est un alcool choisi parmi les alcools primaires, secondaires, les alcools à longue chaîne hydrocarbonée et les alcools aromatiques.

6. Composition selon la revendication 5 telle que la concentration en alcool est comprise entre 0,01% et 20% en poids par rapport au poids total de la composition et de préférence comprise entre 0,05% et 10% en poids par rapport au poids total de la composition.

7. Composition selon l'une des revendications de 1 à 6, telle qu'elle comprend en tant que précurseur de colorant d'oxydation une base d'oxydation classique choisie parmi les paraphénylènediamines, les bis-phénylalkylènediamines, les para-aminophénols, les orthoaminophénols, les bases hétérocycliques et leurs sels d'addition.

8. Composition selon la revendication 7, telle que la concentration de cette base d'oxydation est comprise entre 0,0005 et 12% en poids par rapport au poids total de la composition.

9. Composition selon l'une des revendications de 1 à 8, telle que la composition comprend en tant que précurseur de colorant d'oxydation un coupleur d'oxydation classique choisi parmi les métaphénylénediamines, les métaaminophénols, les métadiphénols, les coupleurs naphtaléniques, les coupleurs hétérocycliques ainsi que leurs sels d'addition.

10. Composition selon la revendication 9, telle que la concentration de ce coupleur est comprise entre 0,0001 et 10 % en poids par rapport au poids total de la composition.

11. Composition selon l'une des revendications 1 à 10, telle que ledit substrat pour l'enzyme est un précurseur de colorant d'oxydation portant au moins une fonction alcool.

12. Composition selon la revendication 11, telle que le précurseur est choisi parmi les bases d'oxydation portant au moins une fonction alcool suivantes les paraphénylènediamines, les bis-phénylalkylènediamines, les para-aminophénols, les orthoaminophénols, les bases hétérocycliques et leurs sels d'addition, et les coupleurs d'oxydation portant au moins une fonction alcool suivants les métaphénylènediamines, les métaaminophénols, les métadiphénols, les coupleurs naphtaléniques et les coupleurs hétérocycliques ainsi que leurs sels d'addition.

13. Composition selon la revendication 12, telle que le précurseur est choisi parmi les bases d'oxydation suivantes la N,N-bis-(β-hydroxyéthyl) paraphénylénediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la N-(β-hydroxypropyl) paraphénylènediamine, la 2-hydroxyméthyl paraphénylènediamine, la N,N-(éthyl, β-hydroxyéthyl) paraphénylènediamine, la N-(β,γ-dihydroxypropyl) paraphénylènediamine, la 2-β-hydroxyéthyloxy paraphénylènediamine, le 2-β hydroxyéthylamino 5-amino toluène, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(β-hydroxyéthyl) N,N'-bis-(4-aminophényl) tétraméthylènediamine, le 4-amino 3-hydroxyméthyl phénol, le 4-amino 2-hydroxyméthyl phénol, le 4-amino 2-(β-hydroxyéthyl aminométhyl) phénol, le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, le 4,5-diamino 1-(β-hydroxyéthyl) pyrazole, le 4,5-diamino 1-(β-hydroxyéthyl) 3-méthyl pyrazole, le 4,5-diamino 3-hydroxyméthyl 1-méthyl pyrazole, le 4,5-diamino 3-hydroxyméthyl 1-isopropyl pyrazole et les coupleurs d'oxydation suivants le 5-N-(β-hydroxyéthyl)amino 2-méthyl phénol, le 2,4-diamino 1-(β-hydroxyéthyloxy) benzène, le 2-amino 4-(β-hydroxyéthylamino) 1-méthoxybenzène, le 1-β-hydroxyéthylamino-3,4-méthylènedioxybenzène.

14. Composition selon l'une des revendications 1 à 13, **caractérisée par le fait qu'**elle renferme un ou plusieurs colorants directs.

15. Composition selon l'une des revendications 7, 9 ou 12, telle que les sels d'addition sont choisis parmi les chlorhydrates, les bromhydrates, les sulfates, les citrates, les succinates, les tartrates, les lactates, les tosylates, les benzènesulfonates, les phosphates, les acétates, les sels d'addition avec la soude, la potasse, l'ammoniaque, les amines ou les alcanolamines.

16. Composition selon l'une quelconque des revendications 1 à 15, telle que le système colorant consiste en au moins un précurseur de colorant d'oxydation portant au moins une fonction alcool et au moins une enzyme alcool oxydase.

17. Composition selon l'une quelconque des revendications 1 à 16, telle qu'elle est une composition prête à l'emploi, stockée sous forme anaérobie, exempte d'oxygène gazeux.

18. Procédé de teinture des fibres kératiniques et en particulier des fibres kératiniques humaines telles que les cheveux, **caractérisé par le fait qu'**on applique sur lesdites fibres au moins une composition telle que définie dans l'une des revendications 1 à 17, pendant une durée suffisante pour développer la coloration désirée.

19. Procédé selon la revendication 18, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, puis à procéder au mélange des compositions (A) et (B) au moment de l'emploi avant d'appliquer ce mélange sur les fibres kératiniques.

20. Dispositif à plusieurs compartiments ou «Kit» de teinture, **caractérisé par le fait qu'**il comporte un premier compartiment renfermant la composition (A) telle que définie dans la revendication 19 et un second compartiment renfermant la composition (B) telle que définie dans la revendication 19.

## Claims

1. Composition for dyeing keratin fibres, and in particular human keratin fibres such as the hair, containing, in a support that is suitable for dyeing, at least one oxidation dye precursor, **characterized in that** it comprises at least one alcohol oxidase enzyme as sole enzyme for the composition, chosen from primary alcohol oxidases (EC1.1.3.13), secondary alcohol oxidases (EC 1.1.3.18), long-hydrocarbon-chain alcohol oxidases (EC 1.1.3.20), polyvinyl alcohol oxidases (EC 1.1.3.30), vanillyl alcohol, oxidase (EC 1.1.3.38) and aromatic alcohol oxidases (EC 1.1.3.7), and at least one substrate for the said enzyme.

2. Composition according to Claim 1, such that the alcohol oxidase enzyme is derived from an extract chosen from extracts of plants, of animals or of micro-organisms (bacterium, fungus, yeast, micro-alga or virus), of differentiated or undifferentiated cells, obtained *in vivo* or *in vitro,* unmodified or genetically modified, or synthetic.

3. Composition according to Claim 2, such that the alcohol oxidase enzyme is chosen from the genera *Pinus, Gastropode, Manduca, Pichia, Candida, Pleurotus* and *Pseudomonas,* and even more particularly from the following species: *Pinus strobus, Gastropode mollusc, Manduca sexta, Pichia pastoris, Candida boidinii, Pleurotus pulmonarius* and *Pseudomonas pseudoalcaligenes.*

4. Composition according to one of Claims 1 to 3, such that the concentration of alcohol oxidase enzyme is between 0.005% and 40% by weight relative to the total weight of the said composition, and preferably between 0.05% and 10% by weight relative to the total weight of the said composition.

5. Composition according to one of Claims 1 to 4, such that the substrate for the enzyme is an alcohol chosen from primary alcohols, secondary alcohols, long-hydrocarbon-chain alcohols and aromatic alcohols.

6. Composition according to Claim 5, such that the alcohol concentration is between 0.01% and 20% by weight relative to the total weight of the composition, and preferably between 0.05% and 10% by weight relative to the total weight of the composition.

7. Composition according to one of Claims 1 to 6, such that it comprises as oxidation dye precursor a standard oxidation base chosen from paraphenylenediamines, bis(phenyl)alkylenediamines, para-aminophenols, ortho-aminophenols and heterocyclic bases, and the addition salts thereof.

8. Composition according to Claim 7, such that the concentration of this oxidation base is between 0.0005% and 12% by weight relative to the total weight of the composition.

9. Composition according to one of Claims 1 to 8, such that the composition comprises as oxidation dye precursor a standard oxidation coupler chosen from meta-phenylenediamines, meta-aminophenols, meta-diphenols, naphthalenic couplers and heterocyclic couplers, and also the addition salts thereof.

10. Composition according to Claim 9, such that the concentration of this coupler is between 0.0001% and 10% by weight relative to the total weight of the composition.

11. Composition according to one of Claims 1 to 10, such that the said substrate for the enzyme is an oxidation dye precursor bearing at least one alcohol function.

12. Composition according to Claim 11, such that the precursor is chosen from the oxidation bases below bearing at least one alcohol function, paraphenylenediamines, bis(phenyl)alkylenediamines, para-aminophenols, ortho-aminophenols and heterocyclic bases, and the addition salts thereof, and the oxidation couplers below bearing at least one alcohol function, meta-phenylenediamines, meta-aminophenols, meta-diphenols, naphthalenic couplers and heterocyclic couplers, and also the addition salts thereof.

13. Composition according to Claim 12, such that the precursor is chosen from the following oxidation bases: N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, N-(β-hydroxypropyl)-para-phenylenediamine, 2-hydroxymethyl-para-phenylenediamine, N-ethyl-N-(β-hydroxyethyl)-para-phenylenediamine, N-(β,γ-dihydroxypropyl)-para-phenylenediamine, 2-β-hydroxyethyloxy-para-phenylenediamine, 2-β-hydroxyethylamino-5-aminotoluene, N,N'-bis-(β-hydroxyethyl)-N,N'-bis(4'-aminophenyl)-1,3-diaminopropanol, N,N'-bis(β-hydroxyethyl)-N,N'-bis-(4'-aminophenyl)ethylenediamine, N,N'-bis-(β-hydroxyethyl)-N,N'-bis(4-aminophenyl)tetramethylenediamine, 4-amino-3-hydroxymethylphenol, 4-amino-2-hydroxymethylphenol, 4-amino-2-(β-hydroxyethylaminomethyl)phenol, 2-(3-aminopyrazolo[1,5-a]pyrimidin-7-ylamino)ethanol, 2-(7-aminopyrazolo[1,5-a]pyrimidin-3-ylamino)-ethanol, 2-[(3-aminopyrazolo[1,5-a]pyrimidin-7-yl)(2-hydroxyethyl)amino]ethanol, 2-[(7-amino-pyrazolo[1,5-a]pyrimidin-3-yl)(2-hydroxyethyl)-amino]ethanol, 4,5-diamino-1-(β-hydroxyethyl)-pyrazole, 4,5-diamino-1-(β-hydroxyethyl)-3-methylpyrazole, 4,5-diamino-3-hydroxymethyl)-1-methylpyrazole, 4,5-diamino-3-hydroxymethyl-1-isopropyl-pyrazole, and the following oxidation couplers: 5-N-(β-hydroxyethyl)amino-2-methylphenol, 2,4-diamino-1-(β-hydroxyethyloxy)benzene, 2-amino-4-(β-hydroxyethylamino)-1-methoxybenzene, 1-β-hydroxyethylamino-3,4-methylenedioxybenzene.

14. Composition according to one of Claims 1 to 13, **characterized in that** it contains one or more direct dyes.

15. Composition according to one of Claims 7, 9 or 12, such that the addition salts are chosen from the hydrochlorides, hydrobromides, sulphates, citrates, succinates, tartrates, lactates, tosylates, benzenesulphonates, phosphates, acetates and the addition salts with sodium hydroxide, potassium hydroxide, ammonia, amines or alkanolamines.

16. Composition according to any one of Claims 1 to 15, such that the colouring system consists of at least one oxidation dye precursor bearing at least one alcohol function and at least one alcohol oxidase enzyme.

17. Composition according to any one of Claims 1 to 16, such that it is a ready-to-use composition, stored in anaerobic form, free of oxygen gas.

18. Process for dyeing keratin fibres, and in particular human keratin fibres such as the hair, **characterized in that** at least one composition as defined in one of Claims 1 to 17 is applied to the said fibres, for a period that is sufficient to develop the desired coloration.

19. Process according to Claim 18, **characterized in that** it includes a preliminary step that consists in separately storing, on the one hand, a composition (A) comprising in a medium that is suitable for dyeing, at least one oxidation dye precursor, and, on the other hand, a composition (B) containing, in a medium that is suitable for dyeing keratin fibres, at least one alcohol oxidase enzyme, the composition (A) and/or the composition (B) containing at least one substrate for the said enzyme, and then in mixing together the compositions (A) and (B) at the time of use before applying this mixture to the keratin fibres.

20. Multi-compartment device or dyeing "kit", **characterized in that** it comprises a first compartment containing the composition (A) as defined in Claim 19 and a second compartment containing the composition (B) as defined in Claim 19.

## Patentansprüche

1. Zusammensetzung zum Färben von Keratinfasern und insbesondere menschlichen Keratinfasern, wie Haaren, die in einem zum Färben geeigneten Medium mindestens ein Farbstoffvorprodukt eines Oxidationsfarbstoffes enthält, **dadurch gekennzeichnet, dass** sie ferner als einziges in der Zusammensetzung enthaltenes Enzym mindestens eine Alkohol-Oxidase, die unter den Oxidasen für primäre Alkohole (EC 1.1.3.13), Oxidasen für sekundäre Alkohole (EC 1.1.3.18), Oxidasen für Alkohole mit langen Kohlenwasserstoffketten (EC 1.1.3.20), Polyvinylalkohol-Oxidasen (EC 1.1.3.30), Vanillylalkohol-Oxidasen (EC 1.1.3.38) und Oxidasen für aromatische Alkohole (EC 1.1.3.7) ausgewählt ist, und mindestens ein Substrat für das Enzym enthält.

2. Zusammensetzung nach Anspruch 1, wobei die Alkohol-Oxidase von einem Extrakt, der unter den Pflanzenextrakten, tierischen Extrakten oder Extrakten aus Mikroorganismen (Bakterie, Pilz, Hefe, Mikroalge oder Virus) ausgewählt ist, von in vivo oder in vitro gewonnenen differenzierten oder undifferenzierten Zellen stammt, die genetisch modifiziert wurden oder nicht, oder synthetisch ist.

3. Zusammensetzung nach Anspruch 2, wobei die Alkohol-Oxidase unter den Gattungen *Pinus, Gastropode, Manduca, Pichia, Candida, Pleurotus und Pseudomonas* und insbesondere unter den folgenden Species ausgewählt ist: *Pinus strobus, Gastropode mollusc, Manduca sexta, Pichia pastoris, Candida boidinii, Pleurotus pulmonarius und Pseudomonas pseudoalcaligenes.*

4. Zusammensetzung nach einem der Ansprüche 1 bis 3, wobei die Konzentration der Alkohol-Oxidase im Bereich von 0,005 bis 40 Gew-%, bezogen auf das Gesamtgewicht der Zusammensetzung, und vorzugsweise im Bereich von 0,05 bis 10 Gew.-%, bezogen auf das Gesamtgewicht der Zusammensetzung, liegt.

5. Zusammensetzung nach einem der Ansprüche 1 bis 4, wobei das Substrat für das Enzym ein Alkohol ist, der unter den primären Alkoholen, sekundären Alkoholen, Alkoholen mit langen Kohlenwasserstoffketten und aromatischen Alkoholen ausgewählt ist.

6. Zusammensetzung nach Anspruch 5, wobei die Konzentration des Alkohols im Bereich von 0,01 bis 20 Gew.-%, bezogen auf das Gesamtgewicht der Zusammensetzung, und vorzugsweise im Bereich von 0,05 bis 10 Gew.-%, bezogen auf das Gesamtgewicht der Zusammensetzung, liegt.

7. Zusammensetzung nach einem der Ansprüche 1 bis 6, wobei sie als Farbstoffvorprodukt eines Oxidationsfarbstoffes eine herkömmliche Oxidationsbase enthält, die unter den *para*-Phenylendiaminen, Bisphenylalkylendiaminen, *para*-Aminophenolen, *ortho*-Aminophenolen, heterocyclischen Basen und deren Additionssalzen ausgewählt ist.

8. Zusammensetzung nach Anspruch 7, wobei die Konzentration der Oxidationsbase im Bereich von 0,0005 bis 12 Gew.-%, bezogen auf das Gesamtgewicht der Zusammensetzung, liegt.

9. Zusammensetzung nach einem der Ansprüche 1 bis 8, wobei die Zusammensetzung als Farbstoffvorprodukt eines Oxidationsfarbstoffes einen herkömmlichen oxidativen Kuppler enthält, der unter den *meta*-Phenylendiaminen, *meta*-Aminophenolen, *meta-*Dihydroxybenzolen, Naphthalinkupplern, heterocyclischen Kupplern und deren Additionssalzen ausgewählt ist.

10. Zusammensetzung nach Anspruch 9, wobei die Konzentration des Kupplers im Bereich von 0,0001 bis 10 Gew.-%, bezogen auf das Gesamtgewicht der Zusammensetzung, liegt.

11. Zusammensetzung nach einem der Ansprüche 1 bis 10, wobei das Substrat für das Enzym ein Farbstoffvorprodukt eines Oxidationsfarbstoffes ist, das mindestens eine Alkoholfunktion aufweist.

12. Zusammensetzung nach Anspruch 11, wobei das Vorprodukt unter den folgenden Oxidationsbasen, die mindestens eine Alkoholfunktion aufweisen, ausgewählt ist: *para*-Phenylendiaminen, Bisphenylalkylendiaminen, *para*-Aminophenolen, *ortho*-Aminophenolen, heterocyclischen Basen und ihren Additionssalzen; und den folgenden Kupplern, die mindestens eine Alkoholfunktion aufweisen: *meta*-Phenylendiaminen, *meta*-Aminophenolen, *meta*-Dihydroxybenzolen, Naphthalinkupplern, heterocyclischen Kupplern und deren Additionssalzen.

13. Zusammensetzung nach Anspruch 12, wobei das Vorprodukt unter den folgenden Oxidationsbasen: N,N-Bis(β-hydroxyethyl)-para-phenylendiamin, 4-N,N-Bis(β-hydroxyethyl)amino-2-methyl-anilin, 4-N,N-Bis(β-hydroxyethyl)amino-2-chlor-anilin, 2-β-Hydroxyethyl-para-phenylendiamin, N-(β-Hydroxypropyl)-para-phenylendiamin, 2-Hydroxymethyl-para-phenylendiamin, N,N-(Ethyl, β-hydroxyethyl)-para-phenylendiamin, N-(β,γ-Dihydroxypropyl)-para-phenylendiamin, 2-β-Hydroxyethyloxy-para-phenylendiamin, 2-β-Hydroxyethylamino-5-amino-toluol, N,N'-Bis(β-hydroxyethyl)-N,N'-bis(4'-aminophenyl)-1,3-diaminopropanol, N,N'-Bis-(β-hydroxyethyl)-N,N'-bis(4'-aminophenyl)-ethylendiamin, N,N'-Bis(β-hydroxyethyl)-N,N'-Bis(4-aminophenyl)-tetramethylendiamin, 4-Amino-3-hydroxymethyl-phenol, 4-Amino-2-hydroxymethyl-phenol, 4-Amino-2-(β-hydroxyethylaminomethyl)-phenol, 2-(3-Amino-pyrazolo-[1,5-a]-pyrimidin-7-ylamino)-ethanol, 2-(7-Amino-pyrazolo-[1,5-a]-pyrimidin-3-ylamino)-ethanol, 2-[(3-Amino-pyrazolo-[1,5-a]-pyrimidin-7-yl)-(2-hydroxy-ethyl)-amino]-ethanol, 2-[(7-Amino-pyrazolo-[1,5-a]-pyrimidin-3-yl)-(2-hydroxy-ethyl)-amino]-ethanol, 4,5-Diamino-1-(β-hydroxyethyl)-pyrazol, 4,5-Diamino-1-(β-hydroxyethyl)-3-methyl-pyrazol, 4,5-Diamino-3-hydroxymethyl-1-methyl-pyrazol, 4,5-Diamino-3-hydroxymethyl-1-isopropyl-pyrazol; und den folgenden oxidativen Kupplern ausgewählt ist: 5-N-(β-Hydroxyethyl)amino-2-methyl-phenol, 2,4-Diamino-1-(β-hydroxyethyloxy)-benzol, 2-Amino-4-(β-Hydroxyethylamino)-1-methoxybenzol, 1-β-Hydroxyethylamino-3,4-methylendioxybenzol.

14. Zusammensetzung nach einem der Ansprüche 1 bis 13, **dadurch gekennzeichnet, dass** sie einen oder mehrere Direktfarbstoffe enthält.

15. Zusammensetzung nach einem der Ansprüche 7, 9 oder 12, wobei die Additionssalze unter den Hydrochloriden, Hydrobromiden, Sulfaten, Citraten, Succinaten, Tartraten, Lactaten, Tosylaten, Benzolsulfonaten, Phosphaten und Acetaten und den Additionssalzen mit Natriumhydroxid, Kaliumhydroxid, Salmiakgeist, Aminen oder Alkanolaminen ausgewählt sind.

16. Zusammensetzung nach einem der Ansprüche 1 bis 15, wobei das Färbesystem aus mindestens einem Farbstoffvorprodukt eines Oxidationsfarbstoffes, der mindestens eine Alkoholfunktion aufweist, und mindestens einer Alkohol-Oxidase besteht.

17. Zusammensetzung nach einem der Ansprüche 1 bis 16, wobei es sich um eine gebrauchsfertige Zusammensetzung handelt, die unter Luftausschluss ohne gasförmigen Sauerstoff aufbewahrt wird.

18. Verfahren zum Färben von Keratinfasern und insbesondere menschlichen Keratinfasern, wie Haaren, **dadurch gekennzeichnet, dass** mindestens eine Zusammensetzung, wie in einem der Ansprüche 1 bis 17 definiert ist, während einer Zeitspanne auf die Fasern aufgebracht wird, die ausreichend ist, um die gewünschte Färbung zu bilden.

19. Verfahren nach Anspruch 18, **dadurch gekennzeichnet, dass** es einen vorbereitenden Schritt umfasst, der darin besteht, einerseits eine Zusammensetzung (A), die in einem zum Färben geeigneten Medium mindestens ein Farbstoffvorprodukt eines Oxidationsfarbstoffes enthält, und andererseits eine Zusammensetzung (B) getrennt voneinander aufzubewahren, die in einem zum Färben von Keratinfasern geeigneten Medium mindestens eine Alkohol-Oxidase enthält, wobei die Zusammensetzung (A) und/ oder die Zusammensetzung (B) mindestens ein Substrat für das Enzym enthalten, und die Zusammensetzungen (A) und (B) bei der Anwendung zu vermischen, bevor das Gemisch auf die Keratinfasern aufgebracht wird.

20. Vorrichtung mit mehreren Abteilungen oder "Kit" zum Färben, **dadurch gekennzeichnet, dass** sie eine erste Abteilung mit der in Anspruch 19 definierten Zusammensetzung (A) und eine zweite Abteilung mit der in Anspruch 19 definierten Zusammensetzung (B) umfasst.
